(19) Europäisches Patentamt
European Patent Office
Office européen des brevets

(11) **EP 4 803 070 A1**

(12) **EUROPEAN PATENT APPLICATION**
published in accordance with Art. 153(4) EPC

(43) Date of publication:
**09.09.2026 Bulletin 2026/37**

(21) Application number: **24885556.1**

(22) Date of filing: **23.10.2024**

(51) International Patent Classification (IPC):
***A61K 8/86*** (2006.01) ***A61K 8/27*** (2006.01)
***A61K 8/29*** (2006.01) ***A61K 8/35*** (2006.01)
***A61K 8/37*** (2006.01) ***A61K 8/40*** (2006.01)
***A61K 8/41*** (2006.01) ***A61K 8/49*** (2006.01)
***A61K 8/73*** (2006.01) ***A61K 8/81*** (2006.01)
***A61Q 17/04*** (2006.01)

(52) Cooperative Patent Classification (CPC):
**A61K 8/27; A61K 8/29; A61K 8/35; A61K 8/37; A61K 8/40; A61K 8/41; A61K 8/49; A61K 8/73; A61K 8/81; A61K 8/86; A61Q 17/04**

(86) International application number:
**PCT/JP2024/037717**

(87) International publication number:
**WO 2025/094783 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.11.2023 JP 2023187696**

(71) Applicants:
- **NOF Corporation**
  **Shibuya-ku**
  **Tokyo 150-6019 (JP)**
- **Iwase Cosfa Co., Ltd.**
  **Osaka-shi, Osaka 541-0045 (JP)**

(72) Inventors:
- **SHOJI, Ryoka**
  **Kawasaki-shi, Kanagawa 210-0865 (JP)**
- **SEKIGUCHI, Koji**
  **Kawasaki-shi, Kanagawa 210-0865 (JP)**
- **KANEKO, Tomohiro**
  **Osaka-shi, Osaka 541-0045 (JP)**

(74) Representative: **impuls legal PartG mbB**
**Goethestraße 21**
**80336 München (DE)**

# (54) SUNSCREEN COSMETIC

(57) This sunscreen cosmetic contains components (A), (B), and (C). (A): 0.1-10 mass% of a polyalkylene glycol derivative represented by formula (I). (B): 2-40 mass% of an ultraviolet protective agent. (C): 0.01-10 mass% of a water soluble thickening agent. (I): Z-{O-(EO)a-(BO)k-H}m (in the formula, Z represents a residue obtained by removing at least one hydroxyl group from a polyhydric alcohol having three or more carbon atoms and having 3-6 hydroxyl groups, m represents an integer of 3-6, EO represents an oxyethylene group, BO represents an oxybutylene group, and EOs and BOs are bonded in a block-like manner. a and k represent the average number of added moles of EO and BO respectively, a is an integer of 1-50, and k is an integer of 1-50. The mass proportion of (EO)a with respect to the total 100 parts by mass of (EO)a and (BO)k is 10-75 parts by mass.)

EP 4 803 070 A1

## Description

### Technical Field

The present invention relates to a sunscreen cosmetic.

### Background Art

Some polymer compounds exhibit a phase transition in response to external stimuli such as heat, pH, light, and water, and active development is being carried out on temperature sensors, separation membranes, adsorbents, drug release agents, water-absorbing materials, water retention agents, humidity control agents, indicator agents, and the like that utilize this phase transition phenomenon. Among the above-described polymer compounds, those that exhibit a phase transition phenomenon in response to stimuli are referred to as stimuli-responsive polymers. As a stimuli-responsive polymer that is sensitive to thermal stimuli, a living polymer of a vinyl ether containing an oxyethylene chain is known. This living polymer has a cloud point, exhibits hydrophilicity in an aqueous solution at or below the cloud point, and exhibits hydrophobicity at a temperature exceeding the cloud point.

PTL 1 discloses that by using a diblock copolymer of a vinyl ether containing an oxyethylene chain and a vinyl ether containing an azobenzene structure, the wetting properties of the surface of a film formed on various general-purpose resin base materials are modified in response to thermal stimuli.

Stimuli-responsive polymers may also be used in cosmetics. For example, PTL 2 discloses a cosmetic with an ultraviolet protection effect improved by moisture and heat, achieved by combining an alkylene oxide derivative having a specific structure, an ultraviolet protection agent, and an oil phase thickener.

PTL 3 discloses a cosmetic that contains a stimuli-responsive polymer responding to pH stimuli due to its carboxy group therein, is water-resistant to weakly acidic sweat or the like in a range of 0°C to 40°C, and can be easily washed off with weakly alkaline soap water or the like.

### Citation List

#### Patent Literature

PTL 1
Japanese Patent Application Laid-Open No. 2005-154603
PTL 2
WO2020/032244
PTL 3
Japanese Patent Application Laid-Open No. 2017-149649

### Summary of Invention

#### Technical Problem

In the related art, various cosmetics such as sunscreen cosmetics are each blended with an ultraviolet absorber and/or an ultraviolet scatterer as an ultraviolet protection agent, and these components have an effect of protecting the skin from harmful ultraviolet rays. However, when a film formed on the skin from a sunscreen cosmetic comes into contact with moisture such as water or sweat, the ultraviolet absorber or the ultraviolet scatterer are washed out from the film, and the ultraviolet protection effect is inevitably reduced. Therefore, in order to prevent the washing out of the ultraviolet absorber or the ultraviolet scatterer, various attempts have been made, such as improving the water resistance of sunscreen cosmetics.

By blending a film-forming agent capable of imparting water resistance, it is possible to prevent the washing out of the ultraviolet protection agent to the outside due to sweat or moisture from the outside. For example, the cosmetics described in PTL 2 whose ultraviolet protection effect is improved by moisture and heat form a film that is resistant to sweat and water and are highly water-resistant.

However, the film formed from a cosmetic having high water resistance cannot be easily removed with a normal cleanser or soap, and a dedicated cleansing agent must be used. On the other hand, the cosmetics described in PTL 3 are said to be able to be washed off with weakly alkaline soap water or the like. However, from the viewpoint of reducing the burden on the skin during washing, it is desirable to provide a cosmetic that exhibits excellent water resistance while still

being easily removable with water.

An object of the present invention is to provide a sunscreen cosmetic that has excellent water resistance and can also be easily washed off with water.

Solution to Problem

Accordingly, the present invention provides the following.

[1] A sunscreen cosmetic containing components (A), (B), and (C) below:

(A) 0.1 to 10% by mass of a polyalkylene glycol derivative represented by formula (I) below:

$$Z\text{-}\{O\text{-}(EO)a\text{-}(BO)k\text{-}H\}m \qquad (I)$$

in which Z represents a residue obtained by removing at least one hydroxyl group from a polyhydric alcohol having 3 to 6 hydroxyl groups and 3 or more carbon atoms, m represents an integer of 3 to 6, EO represents an oxyethylene group, BO represents an oxybutylene group, EO and BO are bonded in blocks, a represents an average number of added moles of EO and is an integer of 1 to 50, k represents an average number of added moles of BO and is an integer of 1 to 50, and a mass proportion of (EO)a based on 100 parts by mass of a sum of amounts of $(EO)_a$ and $(BO)_k$ is 10 to 75 parts by mass;
(B) 2 to 40% by mass of an ultraviolet protection agent; and
(C) 0.01 to 10% by mass of a water-soluble thickener.

Advantageous Effects of Invention

The present invention can obtain a sunscreen cosmetic that has excellent water resistance and yet can be easily washed off with water.

Description of Embodiments

[Sunscreen Cosmetic]

The present invention provides a sunscreen cosmetic.

The cosmetic of the present invention contains a polyalkylene glycol derivative (A), an ultraviolet protection agent (B), and a water-soluble thickener (C).

[(A) Polyalkylene Glycol Derivative]

In the sunscreen cosmetic of the present invention, the component (A) is a polyalkylene glycol derivative represented by formula (I).

$$Z\text{-}\{O\text{-}(EO)_a\text{-}(BO)_k\text{-}H\}_m \qquad (I)$$

The polyalkylene glycol derivative represented by formula (I) acts as a temperature-responsive surface modifier. According to new findings of the present inventors, by appropriately combining this polyalkylene glycol derivative with other components, it is possible to provide a cosmetic that has high water resistance, exhibits improved ultraviolet protection effects upon contact with moisture such as water or sweat compared with immediately after application, and is capable of forming a film that can be easily washed off with water. The surface modifier means a component that, when added in a small amount to a liquid, can impart functionality to the film obtained by application.

In addition, in the present invention, the term "temperature responsiveness (or being temperature-responsive)" refers to a phenomenon in which the hydrophilicity or hydrophobicity of a film formed by applying a cosmetic or the like changes to hydrophilicity or hydrophobicity at a certain temperature, which is separate from the above-described cloud point phenomenon.

In the formula, Z represents a residue obtained by removing at least one hydroxyl group from a polyhydric alcohol having 3 or more carbon atoms and having 3 to 6 hydroxyl groups. Examples of the polyhydric alcohol include glycerin and trimethylolpropane each having three hydroxyl groups, diglycerin, erythritol, pentaerythritol, sorbitan, and methyl glucoside each having four hydroxyl groups, xylitol and triglycerin each having five hydroxyl groups, and sorbitol, inositol, and dipentaerythritol each having six hydroxyl groups. Z is preferably a residue obtained by removing at least hydroxyl group

from an alcohol having 4 to 6 or more hydroxyl groups. From the viewpoint of easily forming a film having high surface uniformity on an uneven base material, enhancing temperature responsiveness, and improving the ultraviolet protection effect after application due to contact with moisture such as water or sweat when formulated into a sunscreen cosmetic (hereinafter, also simply referred to as a "cosmetic"), Z is more preferably a residue obtained by removing at least one hydroxyl group from an alcohol having four hydroxyl groups, particularly preferably a residue obtained by removing at least one hydroxyl group from diglycerin or pentaerythritol, and most preferably a residue obtained by removing at least one hydroxyl group from pentaerythritol.

In the formula, m is 3 to 6, preferably 4 to 6, and more preferably 4. When m is 3 or more, the temperature responsiveness is increased, and the transition between hydrophobicity and hydrophilicity can be easily induced in response to a temperature change. When m is 6 or less, a surface having high uniformity can be easily formed even on an uneven base material. In addition, when m is 3 or more and 6 or less, it is easy to achieve both high water resistance and high ease of removal by washing when formulated into a cosmetic.

In addition, EO represents an oxyethylene group, and BO represents an oxybutylene group, but from the viewpoint of facilitating polymerization, BO is preferably a 1,2-oxybutylene group. In addition, EO and BO are bonded in blocks to facilitate polymerization. Further, a represents the average number of added moles of EO, and k represents the average number of added moles of BO, where a is an integer of 10 to 50, preferably 10 to 40, and particularly preferably 20 to 30, and k is an integer of 3 to 40, preferably 5 to 30, more preferably 10 to 25, and particularly preferably 10 to 15. Further, the mass proportion of $(EO)_a$ is 10 to 75 parts by mass, preferably 15 to 60 parts by mass, more preferably 20 to 60 parts by mass, and particularly preferably 30 to 60 parts by mass based on 100 parts by mass of the sum of the amounts of $(EO)_a$ and $(BO)_k$. By using EO and BO in combination, the temperature responsiveness can be enhanced, and a film having high surface uniformity can be easily formed even on an uneven base material. In addition, by using EO and BO in combination, the water resistance of the film can be enhanced.

In the polyoxyalkylene glycol derivative represented by formula (I) of the present invention, a block of EO and a block of BO are arranged in this order with respect to the residue (Z) obtained by removing at least one hydroxyl group from a polyhydric alcohol. By arranging the blocks in this order, a film having high surface uniformity can be formed.

The polyoxyalkylene glycol derivative represented by formula (I) of the present invention can be produced by a known method. For example, the polyoxyalkylene glycol derivative can be obtained by addition-polymerizing oxyethylene and oxybutylene in this order to a polyhydric alcohol having 3 to 6 hydroxyl groups in the presence of an alkali or acid catalyst.

In the cosmetic of the present invention, the polyalkylene glycol derivative contained as the component (A) imparts water resistance and ease of removal by washing to the film formed from the cosmetic. In addition, upon contact with moisture such as water or sweat, the polyalkylene glycol derivative forms a film with a more uniform surface than before contact, enhancing the ultraviolet protection effect of the film formed from the cosmetic.

The content of the polyalkylene glycol derivative (A) based on the total mass of the cosmetic of the present invention is preferably 0.1% to 10% by mass, more preferably 0.5% to 8% by mass, and still more preferably 1% to 5% by mass. When the content of the polyalkylene glycol derivative is 0.1% by mass or more, the water resistance of the film is enhanced, and the ultraviolet protection ability is improved when the moisture is applied. When the content of the polyalkylene glycol derivative is 10% by mass or less, the film is easily removed by washing.

[(B) Ultraviolet Protection Agent]

In the cosmetic of the present invention, the ultraviolet protection agent contained as the component (B) may be an ultraviolet absorber or an ultraviolet scatterer. The ultraviolet protection agent (B) imparts ultraviolet absorbing or scattering properties to the cosmetic, thereby imparting the protective property of the base material against ultraviolet rays to the cosmetic.

The ultraviolet absorber may be a liquid ultraviolet absorber or a solid ultraviolet absorber.

In the present specification, the term "liquid" refers to a state in which the substance has fluidity under an environment of 25°C at 1 atm, that is, a state under a temperature condition equal to or higher than the melting point (in the case of an amorphous substance without a melting point, a state under at temperature condition equal to or higher than the softening point). In addition, the term "solid" refers to a state in which the substance does not have fluidity under an environment of 25°C at 1 atm, that is, a state under a temperature condition lower than the melting point (in the case of an amorphous substance without a melting point, a state under a temperature condition lower than the softening point).

Examples of the liquid ultraviolet absorber include cinnamic acid-based ultraviolet absorbers such as 2-ethylhexyl p-methoxycinnamate, 2-ethoxyethyl p-methoxycinnamate, a mixture of isopropyl p-methoxycinnamate and diisopropyl cinnamate, and methylbis(trimethylsiloxy)silylisopentyl trimethoxycinnamate; p-aminobenzoic acid-based ultraviolet absorbers such as amyl p-dimethylaminobenzoate and 2-ethylhexyl p-dimethylaminobenzoate; salicylic acid-based ultraviolet absorbers such as ethylene glycol salicylate, 2-ethylhexyl salicylate, benzyl salicylate, and homomenthyl salicylate; octocrylene; and dimethicodiethylbenzalmalonate. From the viewpoint of easily improving the ultraviolet

protection effect of the film after contact with moisture such as water or sweat compared to immediately after application, 2-ethylhexyl p-methoxycinnamate, octocrylene, and dimethicodiethylbenzalmalonate are preferable, and 2-ethylhexyl p-methoxycinnamate and octocrylene are particularly preferable.

Examples of the solid ultraviolet absorber include diethylamino hydroxybenzoyl hexyl benzoate, bis-ethylhexyloxyphenol methoxyphenyl triazine, methylene bis-benzotriazolyl tetramethylbutylphenol, ethylhexyl triazone, 2-ethylhexyl dimethoxybenzylidene dioxoimidazolidine propionate, and t-butyl methoxydibenzoylmethane. From the viewpoint of easily improving the ultraviolet protection effect of the film after contact with moisture such as water or sweat compared to immediately after application, diethylamino hydroxybenzoyl hexyl benzoate, bis-ethylhexyloxyphenol methoxyphenyl triazine, ethylhexyl triazone, and t-butyl methoxydibenzoylmethane are particularly preferable.

From the viewpoint of easily improving the ultraviolet protection effect of the film after contact with moisture such as water or sweat compared to immediately after application, the cosmetic preferably contains at least a liquid ultraviolet absorber, and more preferably contains both a liquid ultraviolet absorber and a solid ultraviolet absorber.

The ultraviolet scatterer is not particularly limited. Specific examples of the ultraviolet scatterer include fine particle metal oxides, such as zinc oxide, titanium oxide, iron oxide, cerium oxide, and tungsten oxide.

The ultraviolet scatterer may be untreated or may be subjected to various hydrophobic surface treatments, but is preferably subjected to a hydrophobic surface treatment. As the surface treatment agent, those commonly used in the cosmetic field, for example, silicones such as dimethicone and alkyl-modified silicones, alkoxysilanes such as octyltriethoxysilane, dextrin fatty acid esters such as dextrin palmitate, and fatty acids such as stearic acid can be used.

From the viewpoint of ultraviolet protection effect, zinc oxide subjected to a hydrophobic surface treatment and titanium oxide subjected to a hydrophobic surface treatment are preferable.

The cosmetic may contain only an ultraviolet absorber, only an ultraviolet scatterer, or both an ultraviolet absorber and an ultraviolet scatterer as the ultraviolet protection agent (B). From the viewpoint of improving the ultraviolet protection effect of the film by contact with moisture such as water or sweat, the cosmetic preferably contains at least an ultraviolet absorber, and more preferably contains both an ultraviolet absorber and an ultraviolet scatterer.

The content of the ultraviolet protection agent (B) based on the total mass of the cosmetic of the present invention is preferably 2% to 40% by mass, more preferably 3% to 38% by mass, and still more preferably 5% to 30% by mass. When the content of the ultraviolet protection agent is 2% by mass or more, the ultraviolet protection ability of the film is enhanced. In addition, when the content of the ultraviolet protection agent is 40% by mass or less, the film is easily removed by washing.

[(C) Water-Soluble Thickener]

In the cosmetic of the present invention, the water-soluble thickener contained as the component (C) is a water-soluble thickener that can be typically used in cosmetics.

Examples of the water-soluble thickener include polysaccharide-based polymers, vinyl-based polymers, polyoxyethylene polyoxypropylene-based polymers, acrylic polymers, inorganic polymers, acrylamide-based polymers, and hydrophobically modified polyether urethanes.

Examples of the polysaccharide-based polymer include cellulose-based thickeners such as crystalline cellulose, methyl cellulose, ethyl cellulose, hydroxyethyl cellulose, hydroxypropyl cellulose, and carboxymethyl cellulose; and natural polymer polysaccharide-based thickeners such as gellan gum, carrageenan, guar gum, locust bean gum, gum arabic, xanthan gum, dextran, and succinoglycan.

Examples of the vinyl-based polymer include polyvinyl methyl ether and carboxyvinyl polymer.

Examples of the polyoxyethylene polyoxypropylene-based polymer include polyoxyethylene polyoxypropylene glycol (150 E.O.) (30 P.O.) and polyoxyethylene polyoxypropylene glycol (240 E.O.) (60 P.O.).

Examples of acrylic polymers include (acrylates/alkyl acrylate (C10-30)) crosspolymer, which is an acrylic acid/alkyl methacrylate copolymer; alkyl acrylate/alkyl methacrylate polyoxyethylene ester copolymers; alkyl acrylate/alkyl itaconate polyoxyethylene ester copolymers; and steareth-10 allyl ether/alkyl acrylate copolymers.

Examples of the inorganic polymer include bentonite, magnesium aluminum silicate, laponite, hectorite, and silicic anhydride.

Examples of acrylamide-based polymers include copolymers of hydroxyethyl acrylate and an acryloyldimethyltaurate; copolymers of an acrylate and an acryloyldimethyltaurate; copolymers of acrylamide and an acrylate; copolymers of acrylic acid, acrylamide, an acrylate, and an acryloyldimethyltaurate; and copolymers of vinylpyrrolidone and an acryloyldimethyltaurate. More specifically, examples thereof include (hydroxyethyl acrylate/sodium acryloyldimethyltaurate) copolymer, (sodium acrylate/sodium acryloyldimethyltaurate) copolymer, (acrylamide/ammonium acrylate) copolymer, (acrylic acid/acryloyldimethyltaurate/dimethylacrylamide) crosspolymer, (ammonium acryloyldimethyltaurate/VP) copolymer, (acrylic acid/acrylamide/sodium acrylate/sodium acryloyldimethyltaurate) copolymer, (ammonium acryloyldimethyltaurate/beheneth-25 methacrylate) crosspolymer, and (ammonium acryloyldimethyltaurate/steareth-25 methacrylate) crosspolymer.

The hydrophobically modified polyether urethane is a polymer having a urethane bond modified with a hydrophobic group, and specific examples thereof include (PEG-240/decyl tetradeceth-20/HDI) copolymer.

Among these, from the viewpoint of improving the ultraviolet protection effect of the film more easily than immediately after application by contact with moisture such as water or sweat, polysaccharide-based polymers, vinyl-based polymers, acrylic polymers, and acrylamide-based polymers are preferable, and polysaccharide-based polymers and acrylamide-based polymers are more preferable.

The content of the water-soluble thickener (C) based on the total mass of the cosmetic of the present invention is preferably 0.01% to 10% by mass, more preferably 0.02% to 7.5% by mass, and still more preferably 0.05% to 5% by mass. When the content of the water-soluble thickener is 0.01% by mass or more, the ultraviolet protection effect of the film by contact with moisture such as water or sweat is more easily improved than immediately after application. When the content of the water-soluble thickener is 10% by mass or less, it is easy to achieve both the water resistance and the ease of removal of the film by washing.

The cosmetic of the present invention may contain other components in addition to the component (A), the component (B), and the component (C). Examples of such components include humectants, thickeners other than the component (C), emulsifiers, oil agents, pH adjusters, surfactants, antioxidants, chelating agents, preservatives, skin whitening ingredients, pigments, colorants, and fragrances.

The cosmetic of the present invention can adopt a dosage form as required. Examples of the dosage form include lotions, dispersions, emulsions, creams, gels, stick shapes, cake types, powder forms, sprays, and aerosols. These formulations can be produced by a conventional method using the component (A), the component (B), the component (C), and other commonly used components. In addition, the cosmetic of the present invention may be used as a makeup base having a sunscreen effect.

Examples

Hereinafter, the present invention will be described in detail with reference to examples, but the present invention is not limited to these examples. The numerical values described in the tables of examples are all in units of % by mass with respect to the entire cosmetic.

[Synthesis of Compounds 1 to 9]

Into an autoclave, 45 g of pentaerythritol, 50 g of toluene, and 8.0 g of potassium hydroxide were charged. After the air in the autoclave was replaced with dry nitrogen, 1292 g of ethylene oxide was added dropwise from a dropping funnel at 110°C while stirring, followed by stirring for 2 hours. Subsequently, 1160 g of 1,2-butylene oxide was added dropwise at 110°C, followed by stirring for 2 hours. The reactant was then taken out from the autoclave, neutralized with hydrochloric acid to a pH of 6 to 7, and subjected to a decompression treatment at 115°C for 1 hour to remove toluene and moisture contained therein. Finally, the mixture was filtered to remove the salt, and 2345 g of compound 1 was synthesized. Compounds 2 to 7 were synthesized by changing the type of polyhydric alcohol as a raw material and the amounts of ethylene oxide and 1,2-butylene oxide.

Furthermore, propylene oxide was used to synthesize compounds 8 and 9 under the same conditions as those for the synthesis of compounds 1 to 7. Compound 8 was synthesized by simultaneously adding ethylene oxide and propylene oxide dropwise to synthesize a random copolymer. In addition, compound 9 was synthesized by simultaneously adding ethylene oxide, 1,2-butylene oxide, and propylene oxide dropwise to synthesize a random copolymer.

[Compound 10]

As compound 10, 4-phenylazobenzoic acid PEG-12 methyl ether was used.

The type of polyhydric alcohol that provides the residue of Z in formula (I), and the values of m, a, and k for compounds 1 to 9 are shown in Table 1. For compounds 8 and 9, the average number of added moles (l) of the oxypropylene group (PO) is also shown. The values of a, k, and l for compounds 1 to 9 were calculated based on the hydroxyl value.

[Table 1]

| Compound | Z(m) | EO(a) | BO(k) | PO(l) | EO parts by ma ss | Addition mode |
|---|---|---|---|---|---|---|
| 1 | Pentaerythritol (4) | 22 | 12 | - | 53 | Block |
| 2 | Glycerin (3) | 25 | 15 | - | 51 | Block |
| 3 | Diglycerin (4) | 10 | 20 | - | 23 | Block |

(continued)

| Compound | Z(m) | EO(a) | BO(k) | PO(l) | EO parts by ma ss | Addition mode |
|---|---|---|---|---|---|---|
| 4 | Sorbitol (6) | 20 | 14 | - | 47 | Block |
| 5 | Diglycerin (4) | 40 | 10 | - | 71 | Block |
| 6 | Diethylene glycol (2) | 20 | 11 | - | 53 | Block |
| 7 | Glycerin (3) | - | 15 | - | 0 | Homo |
| 8 | Diethylene glycol (2) | 30 | - | 20 | 53 | Random |
| 9 | Glycerin (3) | 8 | 3 | 5 | 41 | Random |

The following evaluations were performed on compounds 1 to 10.

[Preparation of Sunscreen Cosmetic]

Compounds 1 to 10 were mixed with the component (A), the component (B), and the component (C) in Tables 1 to 5, an oil agent other than the component (A) and the component (B), an emulsifier, and a lipophilic additive, and each mixture was added to an oil phase prepared by heating to 75°C to 80°C, followed by mixing. An aqueous phase prepared by heating to 75°C to 80°C was added thereto, and the mixture was uniformly mixed with a homogenizer to prepare each of cosmetics 1 to 19. Dodecane (PARAFOL 12, manufactured by Sasol Ltd.) was used as the oil agent.

Commercially available products provided for cosmetic use were used as the component (A), the component (B), the oil agent other than the component (B) and the component (C), the emulsifier, the lipophilic additive, and the hydrophilic additive in the table.

The following evaluations were performed on cosmetics 1 to 19.

[Water Resistance Evaluation of Sunscreen Cosmetic]

SPF measurement was performed on a sample in which each of cosmetics 1 to 19 was applied to a PMMA plate (HELIOPLATE HD6, manufactured by Labsphere, Inc.) at 2 mg/cm$^2$ and then allowed to stand in a dark place for 20 minutes, using an SPF analyzer (UV-2000S, manufactured by Labsphere, Inc.). The measurement was performed at nine points on the plate to obtain [SPF before water bath] from the average value.

Next, the measured plate was immersed in water at 30°C and stirred in the water for 80 minutes (100 rpm with 3-1 motor). Thereafter, the plate was dried for about 60 to 120 minutes until the water droplets on the surface disappeared, and the SPF measurement was performed again to obtain [SPF after water bath]. The rate of change in SPF after water bath (30°C) was obtained from the following equation (II).

Rate of change in SPF after water bath (30°C) = [SPF after water bath]/[SPF before water bath] × 100 (II)

The water resistance was evaluated based on the obtained rate of change in SPF after water bath (30°C) according to the following criteria.

[Evaluation Criteria]

◎: 130% or more
○: 115% or more and less than 130%
△: 100% or more and less than 115%
×: less than 100%

[Evaluation of Cleanability of Sunscreen Cosmetic]

Brilliant Blue (water-soluble blue dye) at 0.05% by mass was mixed with each of cosmetic 1 to cosmetic 19, and 2 mg/cm$^2$ of the mixture was applied to a PMMA plate (HELIOPLATE HD6, manufactured by Labsphere, Inc.), and the sample was left to stand in a dark place for 20 minutes, and then immersed in water at 20°C and stirred in the water for 80 minutes (100 rpm with a 3-1 motor). Thereafter, the sample was dried for about 60 to 120 minutes until the water droplets on

the surface disappeared, and the difference in brightness before and after the water bath in the sample coating portion was measured with a spectrophotometer (CM-2500c, manufactured by Konica Minolta, Inc.).

The cleaning rate was calculated according to the following expression, and the cleanability was evaluated according to the following criteria.

$$\text{Cleaning rate} = (1 - \Delta Ea/\Delta Eb) \times 100$$

$\Delta Ea$ = brightness of PMMA plate surface after water bath
$\Delta Eb$ = brightness of PMMA plate surface before water bath

[Evaluation Criteria]

◎: cleaning rate of 95% or more
○: cleaning rate of 85% or more and less than 95%
△: cleaning rate of 75% or more and less than 85%
×: cleaning rate of less than 75%

The compositions of cosmetics 1 to 19, as well as the evaluation results of water resistance and cleanability, are shown in Tables 2 to 5.

[Table 2]

| | | | Example | | | | |
|---|---|---|---|---|---|---|---|
| Cosmetic | | | 1 | 2 | 3 | 4 | 5 |
| Component (A) | Compound 1 | | 5 | | | | |
| | Compound 2 | | | 5 | | | |
| | Compound 3 | | | | 3 | | |
| | Compound 4 | | | | | 1 | 1 |
| | Compound 5 | | | | | | |
| Component (B) | Ethylhexyl p-methoxycinnamate | Ultraviolet absorber (Liquid) | 7.5 | 8.5 | 7.5 | 7.5 | 7.5 |
| | Octocrylene | | 1.5 | 2.5 | 1.5 | 1.5 | 1.5 |
| | Diethylamino hydroxybenzoyl hexyl benzoate | Ultraviolet absorber (Solid) | 1.5 | 2.5 | 1.5 | 1.5 | 1.5 |
| | t-Butyl methoxydibenzoylmethane | | 1.5 | 2.5 | 1.5 | 1.5 | 1.5 |
| | Ethylhexyl triazone | | 1.5 | 2.5 | 1.5 | 1.5 | 1.5 |
| | Titanium oxide (hydrophobically treated) | Ultraviolet scatterer | 5 | 7.5 | 5 | 5 | |
| | Zinc oxide (hydrophobically treated) | | 5 | 7.5 | 5 | 5 | |
| Component (C) | Xanthan gum | | 0.5 | | | 0.5 | 2.5 |
| | (Sodium acrylate/sodium acryloyldimethyltaurate) copolymer | | 1.5 | 4 | 3 | | |
| | (Acrylates/alkyl acrylate (C10-30)) crosspolymer | | | | | | |
| | Carboxyvinyl polymer | | | | | | |
| Polysorbate 80 | | | | | | 2 | 2 |
| Dodecane | | | 15 | | | | |
| Water | | | Balance | | | | |
| Total | | | 100 | | | | |

(continued)

| Cosmetic | Example 1 | 2 | 3 | 4 | 5 |
|---|---|---|---|---|---|
| Water resistance of sunscreen cosmetic | 136 | 112 | 120 | 125 | 119 |
| | ◎ | △ | ○ | ○ | ○ |
| Cleanability of sunscreen cosmetic | 95 | 80 | 84 | 91 | 90 |
| | ◎ | △ | △ | ○ | ○ |

[Table 3]

| Cosmetic | | | Example 6 | 7 | 8 | 9 | 10 | 11 | 12 |
|---|---|---|---|---|---|---|---|---|---|
| Component (A) | Compound 1 | | | | | | | | |
| | Compound 2 | | | | | | | | |
| | Compound 3 | | | | | | | | |
| | Compound 4 | | 2 | 2 | 2 | 4 | 0.5 | 2 | 2 |
| | Compound 5 | | | | | | | | |
| Component (B) | Ethylhexyl p-methoxvcinnamate | Ultraviolet absorber (Liquid) | 7.5 | 7.5 | | | 7.5 | 7.5 | |
| | Octocrylene | | 1 | 3.0 | | | 1 | 1 | |
| | Diethylamino hydroxybenzoyl hexvl benzoate | Ultraviolet absorber (Solid) | 1 | | 2 | | 1 | 1 | |
| | t-Butyl methoxydibenzoylmethane | | 1 | | 2 | | 1 | 1 | |
| | Ethylhexyl triazone | | 1 | | 2 | | 1 | 1 | |
| | Titanium oxide (hydrophobically treated) | Ultraviolet scatterer | | | | 5 | | | 1.5 |
| | Zinc oxide (hydrophobically treated) | | | | | 5 | | | 1.5 |
| Component (C) | Xanthan gum | | | | | | | | |
| | (Sodium acrylate/sodium acryloyldimethyltaurate) copolymer | | | | | | | | |
| | (Acrylates/alkyl acrylate (C10-30)) crosspolymer | | | | | 0.1 | 0.2 | | 0.5 |
| | Carboxyvinyl polymer | | 0.05 | 0.05 | 0.05 | | 0.1 | 0.01 | |
| Polysorbate 80 | | | 2 | 2 | 2 | 2 | 1 | 3 | 1 |
| Dodecane | | | 15 | | | | | | |
| Water | | | Balance | | | | | | |
| Total | | | 100 | | | | | | |
| Water resistance of sunscreen cosmetic | | | 115 | 110 | 105 | 101 | 107 | 110 | 102 |
| | | | ○ | Δ | Δ | Δ | Δ | Δ | Δ |
| Cleanability of sunscreen cosmetic | | | 90 | 90 | 89 | 88 | 88 | 88 | 92 |
| | | | ○ | ○ | ○ | ○ | ○ | ○ | ○ |

[Table 4]

| | | | Example | | |
|---|---|---|---|---|---|
| Cosmetic | | | 13 | 14 | 15 |
| Component (A) | Compound 1 | | | | |
| | Compound 2 | | | | |
| | Compound 3 | | | | |
| | Compound 4 | | 3 | 8 | |
| | Compound 5 | | | | 4 |
| Component (B) | Ethylhexyl p-methoxycinnamate | Ultraviolet absorber (Liquid) | 10 | 7.5 | 7.5 |
| | Octocrylene | | 2 | 1.5 | 1.5 |
| | Diethylamino hydroxybenzoyl hexyl benzoate | Ultraviolet absorber (Solid) | 2 | 1.5 | 1.5 |
| | t-Butyl methoxydibenzoylmethane | | 2 | 1.5 | 1.5 |
| | Ethylhexyl triazone | | 2 | 1.5 | 1.5 |
| | Titanium oxide (hydrophobically treated) | Ultraviolet scatterer | 10.5 | | |
| | Zinc oxide (hydrophobically treated) | | 10.5 | | |
| Component (C) | Xanthan gum | | | | 0.5 |
| | (Sodium acrylate/sodium acryloyldimethyltaurate) copolymer | | | | |
| | (Acrylates/alkyl acrylate (C10-30)) crosspolymer | | 0.2 | 0.2 | |
| | Carboxyvinyl polymer | | 0.1 | 0.1 | |
| Polysorbate 80 | | | 5 | 1 | 2 |
| Dodecane | | | 15 | | |
| Water | | | Balance | | |
| Total | | | 100 | | |
| Water resistance of sunscreen cosmetic | | | 123 | 118 | 114 |
| | | | ○ | ○ | Δ |
| Cleanability of sunscreen cosmetic | | | 75 | 78 | 80 |
| | | | Δ | Δ | Δ |

[Table 5]

| | | Comparative example | | | |
|---|---|---|---|---|---|
| Cosmetic | | 16 | 17 | 18 | 19 |
| Component (A) | Compound 6 | 3 | | | |
| | Compound 7 | | 3 | | |
| | Compound 8 | | | 3 | |
| | Compound 9 | | | | 3 |

(continued)

| | | | Comparative example | | | |
|---|---|---|---|---|---|---|
| Cosmetic | | | 16 | 17 | 18 | 19 |
| Component (B) | Ethylhexyl p-methoxycinnamate | Ultraviolet absorber (Liquid) | 7.5 | 7.5 | 7.5 | 7.5 |
| | Octocrylene | | 1 | 1 | 1 | 1 |
| | Diethylamino hydroxybenzoyl hexyl benzoate | Ultraviolet absorber (Solid) | 1 | 1 | 1 | 1 |
| | t-Butyl methoxydibenzoylmethane | | 1 | 1 | 1 | 1 |
| | Ethylhexyl triazone | | 1 | 1 | 1 | 1 |
| | Titanium oxide (hydrophobically treated) | Ultraviolet scatterer | | | | |
| | Zinc oxide (hydrophobically treated) | | | | | |
| Component (C) | Xanthan gum | | 0.5 | 0.5 | 0.5 | 0.5 |
| | (Sodium acrylate/sodium acryloyldimethyltaurate) copolymer | | 1.5 | 1.5 | 1.5 | 1.5 |
| | (Acrylates/alkyl acrylate (C10-30)) crosspolymer | | | | | |
| | Carboxyvinyl polymer | | | | | |
| Polysorbate 80 | | | 1 | 1 | 1 | 1 |
| Dodecane | | | 15 | | | |
| Water | | | Balance | | | |
| Total | | | 100 | | | |
| Water resistance of sunscreen cosmetic | | | 90 | 10 | 8 | 5 |
| | | | × | × | × | × |
| Cleanability of sunscreen cosmetic | | | 50 | 95 | 90 | 98 |
| | | | × | ◎ | ○ | ◎ |

As shown in Tables 2 to 5, a cosmetic containing the component (A) to the component (C) was more likely to improve the ultraviolet protection effect by contact with moisture, had high water resistance, and had satisfactory cleanability.

As shown in Table 5, a cosmetic not containing the component (A) had a decreased ultraviolet protection effect due to contact with moisture.

Specifically, in cosmetic 16 (comparative example 1), the value of m of the component (A) was 2, which was below the lower limit, and thus the water resistance of the sunscreen cosmetic was insufficient. In cosmetic 17 (comparative example 2), the component (A) did not have a structure derived from EO, and thus the water resistance of the sunscreen cosmetic material was insufficient. In cosmetic 18 (comparative example 3), the value of m of the component (A) was 2, which was below the lower limit, and the component (A) did not have a structure derived from BO, and the oxyalkylene group had a random structure. Therefore, the water resistance of the sunscreen cosmetic was insufficient. In cosmetic 19 (comparative example 4), the oxyalkylene group structure of the component (A) was not within the scope of the present invention, and the oxyalkylene group structure had a random structure. Therefore, the water resistance of the sunscreen cosmetic was insufficient.

(Formulation examples)

Hereinafter, Tables 6 to 11 show formulation examples of a sunscreen cosmetic, a makeup base, a two-layer sunscreen mist, and a two-layer sunscreen spray as specific formulation examples of a composition containing the temperature-responsive surface modifier according to the present invention. It goes without saying that the embodiments of the present invention are not limited to these formulation examples. In the tables, "PEG" stands for polyethylene glycol, "PPG" stands for polypropylene glycol, "BG" stands for butylene glycol, and "DPG" stands for dipropylene glycol.

[Table 6]

| Formulation example 1: Sunscreen cosmetic | | | Composition/ % by mass |
|---|---|---|---|
| Component (A) | Compound 1 | | 2.00 |
| Component (B) | 2-Ethylhexyl methoxycinnamate | Ultraviolet absorber (Liquid) | 10.00 |
| | Diethylamino hydroxybenzoyl hexyl benzoate | Ultraviolet absorber (Solid) | 2.00 |
| | Bis-ethylhexyloxyphenol methoxyphenyl triazine | | 1.00 |
| | Zinc oxide (hydrophobically treated) | Ultraviolet scatterer | 15.00 |
| Component (C) | (Sodium acrylate/sodium acryloyldimethyltaurate) copolymer | | 0.56 |
| Other components | Ethanol | | 3.00 |
| | Dimethicone | | 3.00 |
| | Hydrogenated polyisobutene | | 3.00 |
| | Isotridecyl isononanoate | | 2.50 |
| | Polyhydroxystearic acid | | 0.50 |
| | PEG-75 stearate | | 1.15 |
| | Cetearyl alcohol | | 0.50 |
| | Glyceryl stearate | | 0.85 |
| | Propanediol | | 3.00 |
| | Isohexadecane | | 0.34 |
| | Polysorbate 80 | | 0.11 |
| | EDTA-2Na | | Appropriate amount |
| | Phenoxyethanol | | Appropriate amount |
| | Water | | Balance |
| | Total | | 100.00 |

[Table 7]

| Formulation example 2: Sunscreen cosmetic | | | Composition/ % by mass |
|---|---|---|---|
| Component (A) | Compound 1 | | 2.00 |
| Component (B) | 2-Ethylhexyl methoxycinnamate | Ultraviolet absorber (Liquid) | 10.00 |
| | Diethylamino hydroxybenzoyl hexyl benzoate | Ultraviolet absorber (Solid) | 2.00 |
| | Bis-ethylhexyloxyphenol methoxyphenyl triazine | | 1.00 |
| | Titanium oxide (hydrophobically treated) | Ultraviolet scatterer | 10.00 |
| Component (C) | (Sodium acrylate/sodium acryloyldimethyltaurate) copolymer | | 0.56 |

(continued)

<table>
<tr><td colspan="2">Formulation example 2: Sunscreen cosmetic</td><td>Composition/ % by mass</td></tr>
<tr><td>Component (A)</td><td>Compound 1</td><td>2.00</td></tr>
<tr><td rowspan="16">Other components</td><td>Ethanol</td><td>3.00</td></tr>
<tr><td>Dimethicone</td><td>3.00</td></tr>
<tr><td>Hydrogenated polyisobutene</td><td>5.00</td></tr>
<tr><td>Isotridecyl isononanoate</td><td>3.00</td></tr>
<tr><td>Isopropyl myristate</td><td>4.00</td></tr>
<tr><td>Polyhydroxystearic acid</td><td>0.50</td></tr>
<tr><td>PEG-75 stearate</td><td>1.38</td></tr>
<tr><td>Cetearyl alcohol</td><td>0.50</td></tr>
<tr><td>Glyceryl stearate</td><td>1.02</td></tr>
<tr><td>Propanediol</td><td>3.00</td></tr>
<tr><td>Isohexadecane</td><td>0.34</td></tr>
<tr><td>Polysorbate 80</td><td>0.11</td></tr>
<tr><td>EDTA-2Na</td><td>Appropriate amount</td></tr>
<tr><td>Phenoxyethanol</td><td>Appropriate amount</td></tr>
<tr><td>Water</td><td>Balance</td></tr>
<tr><td>Total</td><td>100.00</td></tr>
</table>

[Table 8]

<table>
<tr><td colspan="3">Formulation example 3: Sunscreen cosmetic</td><td>Composition/ % by mass</td></tr>
<tr><td>Component (A)</td><td colspan="2">Compound 1</td><td>2.00</td></tr>
<tr><td rowspan="3">Component (B)</td><td>2-Ethylhexyl methoxycinnamate</td><td>Ultraviolet absorber (Liquid)</td><td>10.00</td></tr>
<tr><td>Diethylamino hydroxybenzoyl hexyl benzoate</td><td rowspan="2">Ultraviolet absorber (Solid)</td><td>2.00</td></tr>
<tr><td>Bis-ethylhexyloxyphenol methoxyphenyl triazine</td><td>1.00</td></tr>
<tr><td>Component (C)</td><td colspan="2">(Sodium acrylate/sodium acryloyldimethyltaurate) copolymer</td><td>0.75</td></tr>
</table>

(continued)

| Formulation example 3: Sunscreen cosmetic | | Composition/ % by mass |
|---|---|---|
| Component (A) | Compound 1 | 2.00 |
| Other components | Ethanol | 3.00 |
| | Dimethicone | 2.00 |
| | Hydrogenated polyisobutene | 2.00 |
| | Isotridecyl isononanoate | 2.00 |
| | PEG-75 stearate | 1.15 |
| | Cetearyl alcohol | 0.50 |
| | Glyceryl stearate | 0.85 |
| | Propanediol | 3.00 |
| | Isohexadecane | 0.45 |
| | Polysorbate 80 | 0.15 |
| | EDTA-2Na | Appropriate amount |
| | Phenoxyethanol | Appropriate amount |
| | Water | Balance |
| | Total | 100.00 |

[Table 9]

| Formulation example 4: Makeup base | | | Composition/ % by mass |
|---|---|---|---|
| Component (A) | Compound 1 | | 5.00 |
| Component (B) | 2-Ethylhexyl methoxycinnamate | Ultraviolet absorber (Liquid) | 7.00 |
| | 4-t-Butyl-4'-methoxydibenzoylmethane | Ultraviolet absorber (Solid) | 1.50 |
| | Bis-ethylhexyloxyphenol methoxyphenyl triazine | | 0.50 |
| | Titanium oxide (hydrophobically treated) | Ultraviolet scatterer | 2.50 |
| | Zinc oxide (hydrophobically treated) | | 2.50 |
| Component (C) | Xanthan gum | | 0.05 |

(continued)

| Formulation example 4: Makeup base | | Composition/ % by mass |
|---|---|---|
| Component (A) | Compound 1 | 5.00 |
| Other components | Cyclopentasiloxane | 15.00 |
| | Dimethicone | 5.00 |
| | Beeswax | 1.00 |
| | Hydrogenated polyisobutene | 1.00 |
| | Sodium hyaluronate | 0.01 |
| | Polyquaternium-51 | 0.01 |
| | PEG/PPG/polybutylene glycol-8/5/3 glycerin | 0.50 |
| | BG | 3.00 |
| | DPG | 3.00 |
| | Propanediol | 3.00 |
| | Aluminum hydroxide | Appropriate amount |
| | Stearic acid | Appropriate amount |
| | Tocopherol | 0.05 |
| | Phenoxyethanol | 0.50 |
| | Ethylhexylglycerin | 0.10 |
| | Glyceryl monooleate | 1.00 |
| | PEG-10 dimethicone | 1.00 |
| | Silica | 1.00 |
| | Talc | 3.00 |
| | Pigment grade titanium oxide | 8.00 |
| | Pigment grade iron oxide | 2.00 |
| | Arginine | Appropriate amount |
| | Water | Balance |
| | Total | 100.00 |

[Table 10]

| Formulation example 5: Two-layer sunscreen mist | | | Composition/ % by mass |
|---|---|---|---|
| Component (A) | Compound 1 | | 5.00 |
| Component (B) | 2-Ethylhexyl methoxycinnamate | Ultraviolet absorber (Liquid) | 7.00 |
| | 4-t-Butyl-4'-methoxydibenzoylmethane | Ultraviolet absorber (Solid) | 1.50 |
| | Bis-ethylhexyloxyphenol methoxyphenyl triazine | | 0.50 |
| | Titanium oxide (hydrophobically treated) | Ultraviolet scatterer | 1.00 |
| Component (C) | Xanthan gum | | 0.10 |

(continued)

| Formulation example 5: Two-layer sunscreen mist | | Composition/ % by mass |
|---|---|---|
| Component (A) | Compound 1 | 5.00 |
| Other components | Isopropyl myristate | 34.70 |
| | Hydrogenated polyisobutene | 10.00 |
| | 2-Diethylhexyl sebacic acid | 5.00 |
| | Methylpolysiloxane | 2.00 |
| | Sunflower seed oil | 0.30 |
| | Trimethylsiloxysilicate | 0.20 |
| | Disteardimonium hectorite | 0.10 |
| | PEG-10 dimethicone | 0.10 |
| | Pentylene glycol | 2.00 |
| | Phenoxyethanol | 0.30 |
| | Tocopherol | 0.10 |
| | Fragrance | 0.10 |
| | Water | 30.00 |
| Total | | 100.00 |

[Table 11]

| Formulation example 6: Two-layer sunscreen spray | Composition/ % by mass |
|---|---|
| Components of two-layer sunscreen mist described in formulation example 3 | 50.0 |
| LPG | 50.0 |
| Total | 100.0 |

The present application is entitled to and claims the benefit of Japanese Patent Application No. 2023-187696, filed on November 1, 2023, the disclosure of which including the specification and claims is incorporated herein by reference in its entirety.

Industrial Applicability

The present invention provides a sunscreen cosmetic that has excellent water resistance and can be easily washed off with water.

**Claims**

**1.** A sunscreen cosmetic comprising components (A), (B), and (C) below:

(A) 0.1 to 10% by mass of a polyalkylene glycol derivative represented by formula (I) below:

$$Z\text{-}\{O\text{-}(EO)_a\text{-}(BO)_k\text{-}H\}_m \qquad (I)$$

wherein Z represents a residue obtained by removing at least one hydroxyl group from a polyhydric alcohol having 3 to 6 hydroxyl groups and 3 or more carbon atoms, m represents an integer of 3 to 6, EO represents an oxyethylene group, BO represents an oxybutylene group, EO and BO are bonded in blocks, a represents an average number of added moles of EO and is an integer of 1 to 50, k represents an average number of added moles of BO and is an integer of 1 to 50, and a mass proportion of $(EO)_a$ based on 100 parts by mass of a sum of

amounts of $(EO)_a$ and $(BO)_k$ is 10 to 75 parts by mass;
(B) 2 to 40% by mass of an ultraviolet protection agent; and
(C) 0.01 to 10% by mass of a water-soluble thickener.

## INTERNATIONAL SEARCH REPORT

International application No. **PCT/JP2024/037717**

**A. CLASSIFICATION OF SUBJECT MATTER**

***A61K 8/86***(2006.01)i; ***A61K 8/27***(2006.01)i; ***A61K 8/29***(2006.01)i; ***A61K 8/35***(2006.01)i; ***A61K 8/37***(2006.01)i; ***A61K 8/40***(2006.01)i; ***A61K 8/41***(2006.01)i; ***A61K 8/49***(2006.01)i; ***A61K 8/73***(2006.01)i; ***A61K 8/81***(2006.01)i; ***A61Q 17/04***(2006.01)i

FI: A61K8/86; A61K8/27; A61K8/29; A61K8/35; A61K8/37; A61K8/40; A61K8/41; A61K8/49; A61K8/73; A61K8/81; A61Q17/04

According to International Patent Classification (IPC) or to both national classification and IPC

**B. FIELDS SEARCHED**

Minimum documentation searched (classification system followed by classification symbols)

A61K8/86; A61K8/27; A61K8/29; A61K8/35; A61K8/37; A61K8/40; A61K8/41; A61K8/49; A61K8/73; A61K8/81; A61Q17/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2012-207001 A (NOF CORPORATION) 25 October 2012 (2012-10-25) claim 1, paragraphs [0005], [0013], [0029], table 1 | 1 |
| Y | JP 2023-106211 A (NOF CORPORATION) 01 August 2023 (2023-08-01) claim 1, paragraphs [0004], [0006], [0019]-[0020], tables 1-2, 4-5 | 1 |
| Y | JP 2013-95737 A (NOF CORPORATION) 20 May 2013 (2013-05-20) claim 1, paragraphs [0005]-[0006], [0011], [0013], tables 1-2, 4 | 1 |
| Y | JP 2012-158550 A (NOF CORPORATION) 23 August 2012 (2012-08-23) paragraph [0012] | 1 |
| Y | JP 2022-45491 A (SHISEIDO COMPANY, LTD.) 22 March 2022 (2022-03-22) paragraph [0002] | 1 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 December 2024** | **24 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/037717**

**C. DOCUMENTS CONSIDERED TO BE RELEVANT**

| Category* | Citation of document, with indication, where appropriate, of the relevant passages | Relevant to claim No. |
|---|---|---|
| Y | WO 2018/221606 A1 (SHISEIDO COMPANY, LTD.) 06 December 2018 (2018-12-06) paragraph [0003] | 1 |
| A | JP 2013-180967 A (NOF CORPORATION) 12 September 2013 (2013-09-12) entire text | 1 |
| A | JP 2011-173878 A (NOF CORPORATION) 08 September 2011 (2011-09-08) entire text, all drawings | 1 |
| A | JP 2006-199665 A (NOF CORPORATION) 03 August 2006 (2006-08-03) entire text | 1 |
| A | JP 2016-6029 A (KAO CORPORATION) 14 January 2016 (2016-01-14) entire text | 1 |

Form PCT/ISA/210 (second sheet) (July 2022)

**INTERNATIONAL SEARCH REPORT**
**Information on patent family members**

International application No.
**PCT/JP2024/037717**

| Patent document cited in search report | Publication date (day/month/year) | Patent family member(s) | Publication date (day/month/year) |
|---|---|---|---|
| JP 2012-207001 A | 25 October 2012 | (Family: none) | |
| JP 2023-106211 A | 01 August 2023 | (Family: none) | |
| JP 2013-95737 A | 20 May 2013 | (Family: none) | |
| JP 2012-158550 A | 23 August 2012 | (Family: none) | |
| JP 2022-45491 A | 22 March 2022 | US 2023/0301879 A1<br>paragraph [0002]<br>WO 2022/054823 A1<br>CN 116033942 A | |
| WO 2018/221606 A1 | 06 December 2018 | US 2021/0128445 A1<br>paragraph [0003]<br>EP 3636249 A1<br>CN 110678168 A<br>KR 10-2020-0011434 A | |
| JP 2013-180967 A | 12 September 2013 | (Family: none) | |
| JP 2011-173878 A | 08 September 2011 | WO 2011/093508 A1<br>CN 102741322 A<br>KR 10-2012-0112705 A | |
| JP 2006-199665 A | 03 August 2006 | (Family: none) | |
| JP 2016-6029 A | 14 January 2016 | WO 2015/182240 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005154603 A
- WO 2020032244 A
- JP 2017149649 A
- JP 2023187696 A